# EUROPEAN PATENT APPLICATION

(11) **EP 1 459 779 A1**
(43) Date of publication of application: **22.09.2004**
(21) Application number: 04251346.5
(22) Date of filing: 09.03.2004
(51) Int. Cl.: A61M 16/06

(54) **Nasal interface including ventilation insert**

(30) Priority: 21.03.2003 US 392959
(71) Applicant: Innomed Technologies, Inc., Boca Raton, Florida 33428 (US)
(72) Inventor: Wood, Thomas J., Blackshear, Georgia 31516 (US)
(74) Representative: Thomson, Paul Anthony

(57) **Abstract**

A ventilation interface (500) to be inserted into a nares of a user of the ventilation interface. A ventilation insert (100) includes a cannula (110) connectable to a source of ventilation gas. A nasal insert (120) adapted to be inserted into a first naris of the nares of the user forms a portion of an input gas flow passage (111,112,120,130) from the cannula (110) to a distal end of the nasal insert (120) for supplying the ventilation gas to the user. A seal portion (130) is adapted to engage a portion of the first naris, the seal portion (130) provided adjacent the distal end. A gas output (140) forms a portion of an output gas flow passage (130,120,112,140) from the nasal insert (120) to an exterior of the ventilation interface (500) for channeling a gas expired by the user. At least a portion of the input gas flow passage (111,112,120,130) and at least a portion of the output gas flow passage (130,120,112,140) are configured to produce laminar flow for the gas passing therethrough when the nasal insert (120) is inserted into the first naris.

## Description

### BACKGROUND OF THE INVENTION

### 1. FIELD OF THE INVENTION

The present invention relates to a ventilation interface to be inserted into a nares of a user of the ventilation interface, and more specifically to a ventilation interface including a ventilation insert adapted to be inserted into a first naris of the nares of the user forming a portion of an input gas flow passage for supplying a ventilation gas to the user.

### 2. DISCUSSION OF RELATED ART

Obstructive sleep apnea syndrome (commonly referred to as obstructive sleep apnea, sleep apnea syndrome, and/or sleep apnea) is characterized by repeated, prolonged episodes of cessation of breathing during sleep. During a period of wakefulness, the muscles of the upper part of the throat passage of an individual keep the passage open, thereby permitting an adequate amount of oxygen to flow into the lungs. During sleep, the throat passage is narrowed due to the relaxation of the muscles. In those persons having a relatively normally sized throat passage, the narrowed throat passage remains open enough to continue to permit the adequate amount of oxygen to flow into the lungs. However, in those persons having a relatively smaller sized throat passage, the narrowed throat passage prohibits an adequate amount of oxygen from flowing into the lungs. Additionally, one or more of a nasal obstruction, a relatively large tongue, and/or certain shapes of the palate and/or the jaw of the individual further prohibit the adequate amount of oxygen from flowing into the lungs.

The individual having the above-discussed conditions can stop breathing for one or more prolonged periods of time (e.g., each period of time being 10 seconds or more). The prolonged periods of time during which breathing is stopped, or apneas, are generally followed by sudden reflexive attempts to breathe. The reflexive attempts to breathe are generally accompanied by a change from a relatively deeper stage of sleep to a relatively lighter stage of sleep. As a result, the individual suffering from obstructive sleep apnea syndrome generally experiences fragmented sleep that is not restful. The fragmented sleep results in one or more of excessive and/or inappropriate daytime drowsiness, headache, weight gain or loss, limited attention span, memory loss, poor judgment, personality changes, lethargy, inability to maintain concentration, and/or depression.

It is known to use a ventilation interface to apply a positive pressure to the throat of the individual that suffers from obstructive sleep apnea syndrome, thereby permitting the adequate amount of oxygen to flow into the lungs. In the known ventilation interface, oxygen and/or room air containing oxygen is delivered through the mouth and/or nose of the individual. Known types of positive pressure applied by the known ventilation interface include continuous positive airway pressure (CPAP) in which a positive pressure is maintained in the throat passage throughout a respiratory cycle, bilevel positive airway pressure (BiPAP) in which a relatively high positive pressure is maintained during inspiration and a relatively low positive pressure is maintained during expiration, and intermittent mechanical positive pressure ventilation (IPPV) in which a positive pressure is applied when apnea is sensed (i.e., the positive airway pressure is applied intermittently or non-continuously).

A known ventilation interface for the application of such positive pressures includes a face mask that covers the nose and/or mouth, as well as two nasal pillows that are inserted into corresponding nares of the naris.

The known face mask requires a harness, such as a headband and/or other headgear components, to keep the mask in a desired and an effective position. However, the use of the harness results in a number of disadvantages.

For example, the required harness is generally uncomfortable, particularly when sleeping. Further, the requires harness must provide and maintain a sufficient fluid or gas tight seal between the mask and the face. However, the provision of such a seal often results in undesirable irritation and sores caused by movement of the mask and harness during periods of both wakefulness and sleep. Further, the required seal is generally unable to be maintained when the mask and harness is moved. The mask also generally applies an undesirable pressure to the sinus area that is adjacent to the nose, causing the nares of the nose to narrow, thereby increasing a velocity of flow through the nares and decreasing a pressure against the nasal mucosal walls. The above-discussed combination of increased flow velocity and decreased pressure results in the removal of moisture from the mucosal walls during inspiration, causing an undesirable drying and a burning sensation within the nares. As a result, the individual may remove the mask to alleviate these discomforts, consequently discontinuing the beneficial application of the positive pressure.

The known nasal pillows include pillowed style nasal seals that are pressed against the interior portion of the nares. However, the known nasal pillows require the use of a harness to keep the nasal pillows in a desired and effective position, resulting in disadvantages similar to those of the known face mask.

For these reasons, it is desirable to provide a nasal interface that overcomes one or more of the above-discussed disadvantages.

### SUMMARY OF THE INVENTION

The present invention provides a ventilation interface to be inserted into a nares of a user of the ventilation interface. A ventilation insert includes a cannula connectable to a source of ventilation gas. A nasal insert is adapted to be inserted into a first naris of the nares of the user, the nasal insert forming a portion of an input gas flow passage from the cannula to a distal end of the nasal insert for supplying the ventilation gas to the user. A seal portion is adapted to engage a portion of the first naris, the seal portion provided adjacent the distal end. A gas output forms a portion of an output gas flow passage from the nasal insert to an exterior of the ventilation interface for channeling a gas expired by the user. At least a portion of the input gas flow passage and at least a portion of the output gas flow passage are configured to produce laminar flow for the gas passing therethrough when the nasal insert is inserted into the first naris.

In a further preferred embodiment of the invention, at least one of the nasal insert and the seal portion is sufficiently flexible to be expanded by a positive pressure provided by the ventilation gas.

The present invention further provides a ventilation interface to be inserted into a nares of a user of the ventilation interface. A ventilation insert includes a cannula connectable to a source of ventilation gas. A nasal insert means forms an input gas flow passage for the ventilation gas from the cannula to a first naris of the nares of the user. A sealing means engages a portion of the first naris. A gas output means forms an output gas flow passage for gas expired by the user from the nasal insert means to an exterior of the ventilation interface. At least a portion of the input gas flow passage and at least a portion of the output gas flow passage are configured to produce laminar flow for the gas passing therethrough when the nasal insert means is inserted into the first naris.

### BRIEF DESCRIPTION OF THE DRAWINGS

A more complete appreciation of the present invention, and one or more of the attendant advantages thereof, will be readily ascertained and/or obtained as the same becomes better understood by reference to the following detailed description when considered in connection with the accompanying drawings, wherein:
Figure 1 shows a side view of a ventilation insert of a ventilation interface according to the present invention.
Figure 2 shows a cross sectional view taken along line II-II of Figure 1.
Figure 3 shows a side isometric view of the ventilation insert of Figure 1.
Figure 4 shows a front isometric view of the ventilation insert of Figure 1.
Figure 5 shows a back isometric view of the ventilation insert of Figure 1.
Figure 6 shows a top isometric view of the ventilation insert of Figure 1.
Figure 7 shows a bottom isometric view of the ventilation insert of Figure 1.

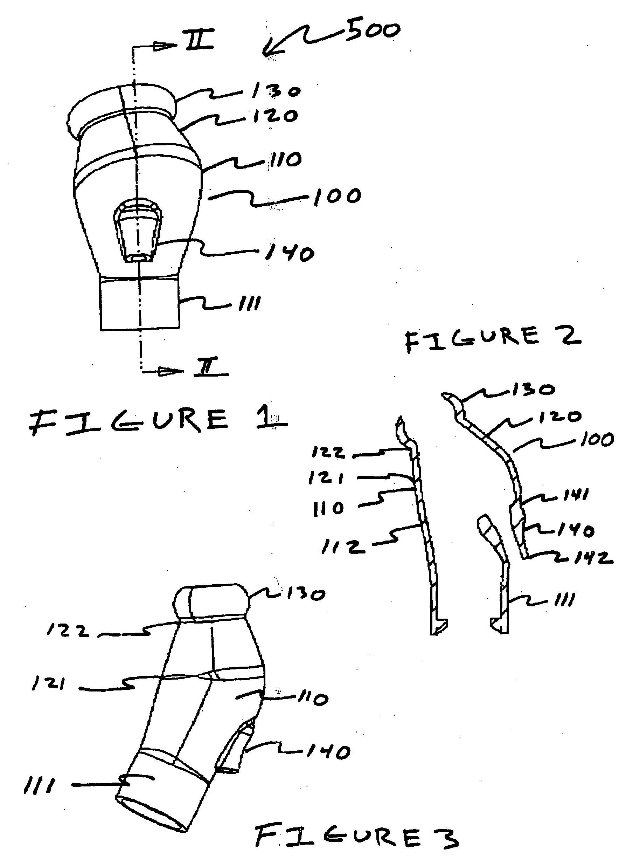

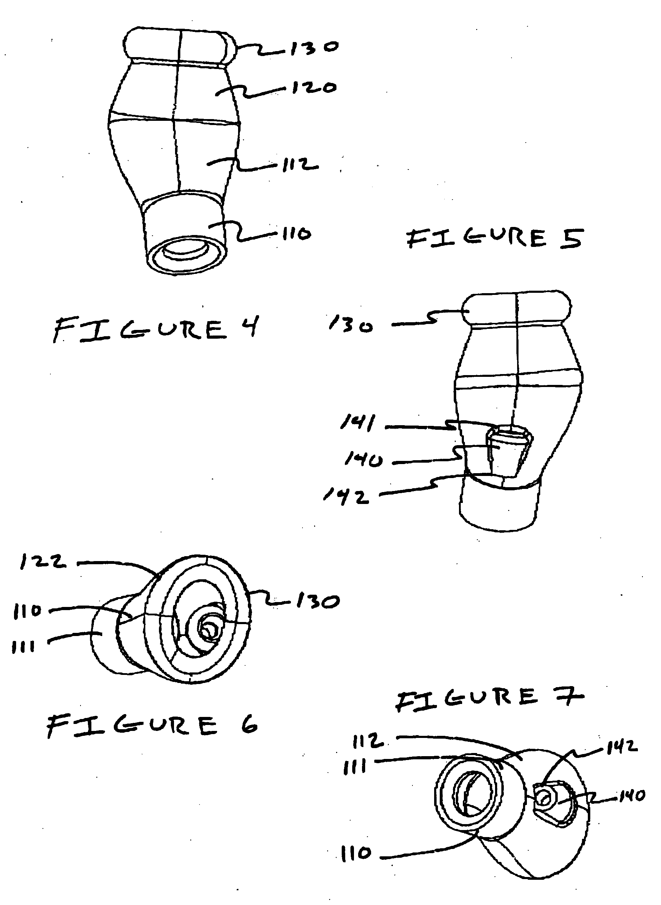

### DETAILED DESCRIPTION OF THE PREFERRED EMBODIMENTS

Examples of one or more preferred embodiments of the present invention will now be described with reference to the drawings, wherein like reference numbers throughout the several views identify like and/or similar elements.

The present invention is directed to a ventilation interface including a first ventilation insert adapted to be inserted into a first naris of a nares of a user forming a portion of a first input gas flow passage for supplying a ventilation gas to the user. A gas output forms a portion of an output gas flow passage from the nasal insert to an exterior of the ventilation interface for channeling a gas expired by the user. At least a portion of the input gas flow passage and at least a portion of the output gas flow passage are configured to produce laminar flow for the gas passing therethrough when the nasal insert is inserted into the first naris. In a preferred embodiment of the invention, a second ventilation insert adapted to be inserted into a second naris of the nares of the user forms a portion of a second input gas flow passage for supplying the ventilation gas to the user that is distinct from the input gas flow passage of the first ventilation insert.

Specifically, as shown in the figures, a ventilation interface 500 according to the present invention can include a first ventilation insert 100. It is to be understood that the ventilation interface 500 can include a second ventilation insert of any type, including of a type that is similar or dissimilar to the first ventilation insert 100, so long as the second ventilation insert defines an input gas flow passage for supplying a ventilation gas to a user of the ventilation interface 500 that is distinct from an input gas flow passage for supplying the ventilation gas to the user defined by the first ventilation insert 100.

The first ventilation insert 100 of the present invention can include, among other components, a cannula 110, a nasal insert 120, a seal portion 130, and a gas output 140, to be described in detail below. The first ventilation insert 100 can include a input gas flow passage adapted to deliver the ventilation gas to the nares of the user of the ventilation interface 500, and more specifically to deliver the ventilation gas from the ventilation source to the interior of the first naris of the nares (described in detail below). In the preferred embodiment of the invention shown in the figures, the input gas flow passage includes first through firth portions through, in part, the cannula 110, the nasal insert 120, and the seal portion 130, respectively. Laminar flow preferably can be achieved within the input gas flow passage. The first ventilation insert 100 can include an output gas flow passage adapted to channel an expired gas by the user to an exterior of the ventilation interface 500, and more specifically to channel the expired gas from the interior of the first naris to the exterior of the first ventilation insert 100 of the ventilation interface 500 (described in detail below). In the preferred embodiment of the invention shown in the figures, the output gas flow passage includes first through sixth portions through, in part, the seal portion 130, nasal insert 120, the cannula 110, and the gas output 140, respectively. Laminar flow preferably can be achieved within the output gas flow passage.

As exemplified in the figures, the cannula 110 can be adapted to be connected to the source of the ventilation gas (not shown), such oxygen and/or room air containing oxygen, to apply a positive pressure to the throat of the user of the ventilation interface 500 that suffers from obstructive sleep apnea syndrome, for example, thereby permitting an adequate amount of oxygen to flow into the lungs. Although the figures show certain preferred embodiments of the cannula 110, it is to be understood that the cannula 110 can be of any type, so long as the cannula 110 can be connected to the source of the ventilation gas.

The cannula 110 can include a connector 111 connectable to the source of the ventilation gas that defines a first portion of the input gas now passage, and more specifically can define the first portion of the input gas flow passage from the source of the ventilation gas through the connector 111. In a preferred embodiment of the invention, the connector 111 can include an exterior surface and an interior surface defining a wall portion therebetween, each of the exterior and interior surfaces having an about circular cross section, such that the connector 10 can define the above-identified flow passage. The interior surface preferably can have a diameter of about 5/16 inch. By this arrangement, the connector 111 can be connected with the source of the ventilation gas by way of a known or conventional tube. Further, the interior surface preferably can include an annular protrusion having a minimum diameter that is less than a diameter of an adjacent portion of the interior surface, such that the connector 111 can be connected with the known or conventional tube that includes a recess corresponding to the annular protrusion. The connector 111 can also extend along an axis such that laminar flow can be achieved within the first portion of the input gas flow passage, and preferably can extend along an axis that is substantially a straight line.

The cannula 110 can include a cannula body 112 adjacent the connector 111 that defines a second portion of the input gas flow passage, and more specifically can define the second portion of the input gas flow passage from the connector 111 through the cannula body 112. The cannula body 112 can define a fourth portion of the output gas flow passage, and more specifically can define the fourth portion of the output gas flow passage from the nasal insert 120 through the cannula body 112. In a preferred embodiment of the invention, the cannula body 112 can include an exterior surface and an interior surface defining a wall portion therebetween, each of the exterior and interior surfaces having a varying cross section including oval and circular shapes, such that the cannula body 112 can define the above-discussed flow passages. The cannula body 112 can extend along one or more axes such that laminar flow can be achieved within the second portion of the input gas flow passage and/or such that laminar flow can be achieved within the fourth portion of the output gas flow passage. Further, the cannula body 112 can extend along the one or more axes such that laminar flow can be achieved between the second portion of the input gas flow passage and portions of the input gas flow passage upstream and/or downstream of the second portion, and/or can extend along the one or more axes such that laminar flow can be achieved between the fourth portion of the output gas flow passage and portions of the output gas flow passage upstream and/or downstream of the fourth portion. Preferably, portions of the axes of the second portion of the input gas flow passage and/or the fourth portion of the output gas flow passage extend along a substantially straight line.

The nasal insert 120 can be disposed adjacent the cannula body 112 and can be adapted to be inserted into the first naris of the nares of the user of the ventilation interface 500 to apply the positive pressure to the throat of the user. Although the figures show certain preferred embodiments of the nasal insert 120, it is to be understood that the nasal insert 120 can be of any type, so long the nasal insert 120 can be inserted into the nares to apply the positive pressure.

The nasal insert 120 can define a third portion and a fourth portion of the input gas flow passage, and more specifically can define the third and fourth portions of the input gas flow passage from the cannula 110 through the nasal insert 120. The nasal insert 120 can define a second portion and a third portion of the output gas flow passage, and more specifically can define the second and third portions of the output gas flow passage from the sealing portion 140 through the nasal insert 120. In a preferred embodiment of the invention, the nasal insert 120 can include an exterior surface and an interior surface defining a wall portion therebetween, each of the exterior and interior surfaces having a varying cross section including oval shapes, such that the nasal inserts 120 can define the above-identified flow passages.

The nasal insert 120 can have a maximum interior cross sectional area and circumference at a proximal end 121 of the nasal insert 120 that is adjacent the cannula 110, thereby defining the third portion of the input gas flow passage, and thereby defining the third portion of the output gas flow passage. The nasal insert 120 can have a minimal interior cross sectional area and circumference at a distal end 122 of the nasal insert 120 that is disposed away from the cannula 110 (i.e., opposite the proximal end 121), thereby defining me fourth portion of the input gas flow passage, and thereby defining the second portion of the output gas flow passage. In a preferred embodiment of the invention, the proximal end 121 can be in the form of an ellipse having a major axis of about 0.875 and a minor axis of about 0.650. The distal end 122 can be in the form of an ellipse having a major axis of about 0.500 and a minor axis of about 0.375.

The nasal insert 120 can extend along an axis such that laminar flow can be achieved within and/or between the third and fourth portions of the input gas flow passage, and/or within and/or between the second and third portions of the output gas flow passage. Further, the nasal insert 120 can extend along the axis such that laminar flow can be achieved among the third and fourth portions of the input gas flow passage and portions of the input gas flow passage upstream and/or downstream of the third and fourth portions, and/or such that laminar flow can be achieved among the second and third portions of the output gas flow passage and portions of the output gas flow passage upstream and/or downstream of the second and third portions. Preferably, the axis is substantially a straight line.

The seal portion 130 can be disposed adjacent the distal end 122 of the nasal insert 120, and can be adapted to engage the interior portion of the first naris to thereby retain the first ventilation insert 100 therewithin. Although the figures show certain preferred embodiments of the seal portion 130, it is to be understood that the seal portion 130 can be of any type, so long as the seal portion 130 can engage the interior portion of the nares.

The seal portion 130 can define a fifth portion of the input gas flow passage, and more specifically can define the fifth portion of the input gas flow passage from the nasal insert 120 through the seal portion 130. The seal portion 130 can define a first portion of the output gas flow passage, and more specifically can define the first portion of the output gas flow passage from the interior of the first naris through the seal portion 130.

In a preferred embodiment of the invention, the seal portion 130 can include an exterior surface and an interior surface defining a wall portion therebetween, each of the exterior and interior surfaces having an about C-shaped or arcuate surface and having an oval cross section, such that the seal portion 130 can define the above-identified flow passages. An interior portion of the seal portion 130 can have an interior cross section in the form of an ellipse having a major axis of about 0.650 and a minor axis of about 0.500. Alternately, as viewed from the top the seal portion 130 can have one or both of an interior surface and an exterior surface with an about tear-shaped cross-section. In a preferred embodiment of the invention, the maximum interior cross section and its corresponding circumference are greater than the interior cross section and circumference of the distal end 122 of the nasal insert 120. A maximum exterior cross section and its corresponding circumference of the seal portion 130 preferably are greater than the exterior cross section and circumference of the distal end 122. In a preferred embodiment of the invention, the seal portion 130 and/or the nasal insert 120 can include an elastic material, the expansion of which retains the first ventilation insert 100 in the nares. Further, the seal portion 130 and/or the nasal insert 120 preferably can be expanded by the positive pressure of the ventilation gas, thereby aiding in the retention of the first ventilation interface 100 within the nares.

The seal portion 130 can extend along an axis such that laminar flow can be achieved within the fifth portion of the input gas flow passage, and/or within the first portion of the output gas flow passage. Further, the seal portion 130 can extend along the axis such that laminar flow can be achieved between the fifth portion of the input gas flow passage and upstream of the input gas flow passage, and/or such that laminar flow can be achieved between the first portion of the output gas flow passage and downstream of the output gas flow passage. Preferably, the axis is substantially a straight line.

The gas output 140 can be disposed adjacent the cannula body 112 opposite to the nasal insert 120, and can be adapted to channel the gas expired by the user to the exterior of the first ventilation insert 100 of the ventilation interface 500. Although the figures show certain preferred embodiments of the gas output 140, it is to be understood that the gas output 140 can be of any type, so long as the gas output 140 can channel the gas expired by the user to an exterior of the ventilation interface 500.

The gas output 140 can define a fifth portion and a sixth portion of the output gas flow passage, and more specifically can define the fifth and sixth portions of the output gas flow passage from the cannula body 112 through the gas output. In a preferred embodiment of the invention, the gas output 140 can include an exterior surface and an interior surface defining a wall portion therebetween, each of the exterior and interior surfaces having a varying cross section including circular shapes, such that the gas output 140 can define the above-identified flow passages. The gas output 140 can have a maximum interior cross sectional area and circumference at a proximal end 141 of the gas output 140 that is adjacent the cannula body 112, thereby defining the fifth portion of the output gas flow passage. The gas output 140 can have a minimal interior cross sectional area and circumference at a distal end 142 of the gas output 140 that is disposed away from the cannula body 112 (i.e., opposite the proximal end 141), thereby defining the sixth portion of the output gas flow passage.

An interior portion of the proximal end 141 can be in the form of a circle having a diameter of about 5mm. An interior portion of the distal end 142 can be in the form of circle having a diameter of about 3mm. In a preferred embodiment of the invention, the interior cross sectional area of the distal end 142 is about 25% of an interior cross sectional area of the sealing portion 130. The gas output 140 can extend along an axis such that laminar flow can be achieved within and/or between the fifth and sixth portions of the output gas flow passage. Further, the gas output 140 can extend along the axis such that laminar flow can be achieved among the fifth and sixth portions of the output gas flow passage and portions of the output gas flow passage upstream of the fifth and sixth portions. Preferably, the axis is substantially a straight line. The gas output 140 can include a reservoir portion adjacent the cannula 110 for retaining a portion of the exhaled gas before flowing to the exterior of the ventilation interface 500.

As discussed above, the input gas flow passage can deliver the ventilation gas to the nares of the user of the ventilation interface 500, and more specifically can deliver the ventilation gas from the ventilation source to and through the first portion of the connector 111 of the cannula 100, to and through the second portion of the cannula body 112 of the cannula, to and through the third portion of the proximal end 121 of the nasal insert 120, to and through the fourth portion of the distal end 122 of the nasal insert 120, to and through the fifth portion of the seal portion 130, to the interior of the first naris of the nares uf the user of the first ventilation insert 100 of the ventilation interface 500. In a preferred embodiment of the invention, laminar flow is achieved among and/or within one or more of the portions (i.e., the first through fifth portions) of the input gas flow passage of the first ventilation insert 100 of the ventilation interface 500, and more preferably is achieved among and within all of the portions of the input gas flow passage.

Also as discussed above, the output gas flow passage can channel the expired gas from the nares of the user of the ventilation interface 500, and more specifically can channel the expired gas from the interior of the first naris of the nares of the user to and through the first portion of the sealing portion 130, to and through the second portion of the distal end 122 of the nasal insert 120, to and through the third portion of the proximal end 121 of the nasal insert 120, to and through the fourth portion of the cannula body 112 of the cannula 110, to and through the fifth portion of the proximal end 141 of the gas output 140, to and through the sixth portion of the distal end 142 of the gas output 140, to the exterior of the first ventilation insert 100 of the ventilation insert 500. In a preferred embodiment of the invention, laminar flow is achieved among and/or within each of the portions (i.e., the first through sixth portions) of the output gas flow passage of the first ventilation insert 100 of the ventilation interface 500, and more preferably is achieved among and within all the portions of the output gas flow passage,

As discussed above, the second ventilation insert of the ventilation interface 500 can be of any type, so long as the second ventilation insert defines an input gas flow passage for supplying ventilation gas to the user of the ventilation interface 100 that is distinct from the input gas flow passage for supplying ventilation gas to the user of the ventilation interface 100 defined by the first ventilation insert 100. In a preferred embodiment of the invention, the second ventilation insert is at least similar to the first ventilation insert 100. Preferably, the first ventilation insert 100 is distinct from the second ventilation insert, such that the first ventilation insert 100 and second ventilation insert are not directed connected with one another (e.g., the first ventilation insert 100 includes a cannula 110 that is separate and distinct from a cannula of the second ventilation insert).

A ventilation interface 500 according to the present invention can avoid disadvantages of the known or conventional ventilation interface requiring a harness. Further, because the ventilation interface 500 does not require the first and second ventilation inserts to be connected to a same cannula, an interior cross sectional area of the first and second ventilation inserts can be increased as compared to the conventional inserts of the conventional ventilation interface. Thus, the ventilation interface 500 according to the present invention can provide the ventilation gas from the ventilation source to the nares at a lower velocity as compared to the conventional ventilation interface, thereby decreasing an amount of moisture removed from the mucosal walls.

Numerous additional modifications and variations of the present invention are possible in light of the above teachings. It is therefore to be understood that within the scope of the appended claims, the present invention may be practiced otherwise than as specifically described herein.

## Claims

1. A ventilation insert of a ventilation interface to be inserted into a nares of a user of the ventilation interface, comprising:
a cannula connectable to a source of ventilation gas;
a nasal insert adapted to be inserted into a first naris of the nares of the user, the nasal insert forming a portion of an input gas flow passage from the cannula to a distal end of the nasal insert for supplying the ventilation gas to the user;
a seal portion adapted to engage a portion of the first naris, the seal portion provided adj acent the distal end; and
a gas output forming a portion of an output gas flow passage from the nasal insert to an exterior of the ventilation interface for channeling a gas expired by the user,
wherein at least a portion of the input gas flow passage and at least a portion of the output gas flow passage are configured to produce laminar flow for the gas passing therethrough when the nasal insert is inserted into the first naris.

2. The ventilation insert according to claim 1, wherein the distal end of the nasal insert defines a first portion of the input gas flow passage having a substantially oval cross section.

3. The ventilation insert according to claim 2, wherein a portion of the nasal insert proximal the cannula defines a second portion of the input gas flow passage having a substantially oval cross section.

4. The ventilation insert according to claim 3, wherein the first portion of the input gas flow passage has a circumference that is less than a circumference of the second portion of the input gas flow passage.

5. The ventilation insert according to claim 4, wherein the seal portion defines a third portion of the input gas flow passage having a substantially oval cross section.

6. The ventilation insert according to claim 5, wherein the second portion of the input gas flow passage has a circumference that is less than a circumference of the third portion of the input gas flow passage.

7. The ventilation insert according to claim 6, wherein the distal end of the nasal insert includes a first exterior portion having a substantially oval cross section.

8. The ventilation insert according to claim 7, wherein the portion of the nasal insert proximal the cannula includes a second exterior portion having a substantially oval cross section.

9. The ventilation insert according to claim 8, wherein the first exterior portion has a circumference that is less than a circumference of the second exterior portion.

10. The ventilation insert according to claim 9, wherein the seal portion includes a third exterior portion having a substantially oval cross section.

11. The ventilation insert according to claim 10, wherein the third exterior portion has a circumference that is greater than a circumference of the second exterior portion.

12. The ventilation insert according to claim 11, wherein the third exterior portion has an about arcuate surface.

13. The ventilation insert according to claim 12, wherein the seal portion has an about arcuate cross section.

14. The ventilation insert according to claim 13, wherein the cannula defines a fourth portion of the input gas flow passage, and the first and second portions defined by the nasal insert are aligned with the fourth portion of the input gas flow passage to provide the laminar flow.

15. The ventilation insert according to claim 14, wherein a distal end of the gas output defines a first portion of the output gas flow passage having a substantially circular cross section.

16. The ventilation insert according to claim 15, wherein a portion of the gas output proximal the cannula defines a second portion of the output gas flow passage having a substantially circular cross section.

17. The ventilation insert according to claim 16, wherein the first portion of the output gas flow passage has a circumference that is less than a circumference of the second portion of the output gas flow passage.

18. The ventilation insert according to claim 17, wherein a portion of the gas output between the first and second portions of the output gas flow passage defines a third portion of the output gas flow passage having a substantially circular cross section and a circumference that is about equal to the circumference of the first portion of the output gas passage defined by the distal end of the gas output.

19. The ventilation insert according to claim 18, wherein the cannula defines a fourth portion of the output gas flow passage, and the first, second, and third portions defined by the gas output are aligned with the fourth portion of the output gas flow passage to provide the laminar flow.

20. The ventilation insert according to claim 19, wherein a direction of flow in the input gas flow passage is about opposite to a direction of flow in the output gas flow passage.

21. The ventilation insert according to claim 20, wherein the third portion of the input gas flow passage defined by the sealing portion has a cross sectional area that is greater than a cross sectional area of the first portion of the output gas flow passage defined by the gas output.

22. The ventilation insert according to claim 21, wherein the cross sectional area of the third portion of the input gas flow passage defined by the sealing portion is equal to about 200% the cross sectional area of the first portion of the output gas flow passage defined by the gas output.

23. The ventilation insert according to claim 22, wherein at least one of the nasal insert and the seal portion is sufficiently flexible to be expanded by a positive pressure provided by the ventilation gas.

24. A ventilation insert of a ventilation interface to be inserted into a nares of a user of the ventilation interface, comprising:
a cannula connectable to a source of ventilation gas;
a nasal insert means for forming an input gas flow passage for the ventilation gas from the cannula to a first naris of the nares of the user;
a sealing means for engaging a portion of the first naris; and
a gas output means for forming an output gas flow passage for gas expired by the user from the nasal insert means to an exterior of the ventilation interface,
wherein at least a portion of the input gas flow passage and at least a portion of the output gas flow passage are configured to produce laminar flow for the gas passing therethrough when the nasal insert means is inserted into the first naris.

25. The ventilation interface according to claim 24, wherein at least one of the nasal insert means and the sealing means is sufficiently flexible to be expanded by a positive pressure provided by the ventilation gas.
